# EUROPÄISCHE PATENTSCHRIFT

(11) **EP 0 485 326 B1**
(45) Veröffentlichungstag und Bekanntmachung des Hinweises auf die Patenterteilung: **25.01.1995**
(21) Anmeldenummer: 91810779.8
(22) Anmeldetag: 04.10.1991
(51) Int. Cl.: A61F 2/36, A61F 2/38

(54) **Zementfrei einbaubare Kappe für einen Femurkopf**
Shell for a femoral head which can be fixed without cement
Calotte de tête fémorale fixable sans ciment

(30) Priorität: 09.11.1990 CH 3567/90
(43) Veröffentlichungstag der Anmeldung: 13.05.1992
(73) Patentinhaber: SULZER Medizinaltechnik AG, 8404 Winterthur (CH); PROTEK AG, 3110 Münsingen-Bern (CH)
(72) Erfinder: Wagner, Heinz, Prof. Dr. med, Orthopädische Klinik, W-8501 Schwarzenbruck (DE); Willi, Roland, CH-8413 Neftenbach (CH)
(74) Vertreter: Trieblnig, Adolf

(56) Entgegenhaltungen:
- EP-A- 0 017 930
- DE-A- 2 751 537
- DE-C- 923 383
- FR-A- 2 225 141
- US-A- 4 846 841

## Beschreibung

Die Erfindung betrifft eine zementfrei einbaubare Kappe für einen Femurkopf, bestehend aus einem Ausschnitt von einer Kugelschale, deren äussere Fläche mindestens einer halbkugelförmig zusammenhängenden Fläche entspricht, vgl. FR-A-2 225 141.

Kappen für Femurköpfe, die die zerstörte Lauffläche zum Gelenk ersetzen, sind vor allem bei jüngeren Patienten sinnvoll, um möglichst viel vom Knochengewebe funktionstüchtig zu erhalten. Der Femurkopf wird als Aufnahmestumpf auf eine der Innenform der Kappe entsprechende Form mechanisch vorbearbeitet und die Kappe wird aufgeschoben. So zeigen die Patentschriften US 3,053,251 und US 3,521,302 Kappen für Femurköpfe, deren primäre Verankerung auf dem Femurkopf den Femurhals gegenüber den hauptsächlichen Biegebelastungen schwächt oder den Markraum verletzt, während für eine dauernde Verankerung wenig Gewähr vorhanden ist. Diesen Umständen trägt die Erfindung Rechnung. Sie löst die Aufgabe, eine gegen die Gelenkmomente wirksame Primärverankerung der Kappe vorzunehmen und die Kappe gegen Abziehkräfte in Richtung ihrer Polachse zu sichern. Gemäss der Erfindung wird die Aufgabe dadurch gelöst,
dass die äussere Oberfläche aus einer verschleissfesten Kobaltlegierung besteht, während die innere am Knochengewebe angreifende Fläche aus Titan besteht und zu einem selbstschneidenden Innengewinde mit konischem Einlauf geformt ist.

Die Vorteile der Erfindung sind darin zu sehen, dass bei der Primärverankerung eine Verdichtung des mit der Kappeninnenseite in Berührung stehenden Knochengewebes stattfindet, und dass auf die Kappe wirkende Belastungskräfte nicht nur als Normalkräfte, sondern auf eine grosse Fläche des Knochengewebes auch in Form von Reibungskräften verteilt werden. Die abhängigen Ansprüche 2 und 3 beziehen sich auf vorteilhafte Weiterbildungen der Erfindung.

Im folgenden wird die Erfindung anhand eines Ausführungsbeispiels beschrieben. Es zeigen:
- Fig. 1: schematisch den vergrösserten Längsschnitt durch eine Femurkopfkappe mit selbstschneidem Innengewinde und mit konischem Einlauf und
- Fig. 2: schematisch in kleinerem Massstab den Längsschnitt durch eine Kappe gemäss Fig. 1, die auf dem Femurknochen befestigt ist.

Die Figuren zeigen eine zementfrei einbaubare Kappe für einen Femurkopf, die aus einem Ausschnitt von einer Kugelschale besteht. Die äussere Fläche entspricht mindestens einer halbkugelförmig zusammenhängenden Fläche, um ohne Unstetigkeiten in der Oberfläche Kräfte bei verschiedenen Gelenkstellungen übertragen zu können.

Erfindungsgemäss besteht die äussere Fläche aus einer verschleissfesten Kobaltlegierung, während die innere, am Knochengewebe angreifende Fläche aus Titan besteht und zu einem selbstschneidenden Innengewinde mit konischem Einlauf geformt ist. Beim Aufgewinden der Kappe wird das sie berührende Knochengewebe einschliesslich der Stirnfläche des vorbearbeiteten Femurkopfs beim Blockieren der Drehbewegung durch das Auftreffen der gewölbten Innenfläche verspannt.

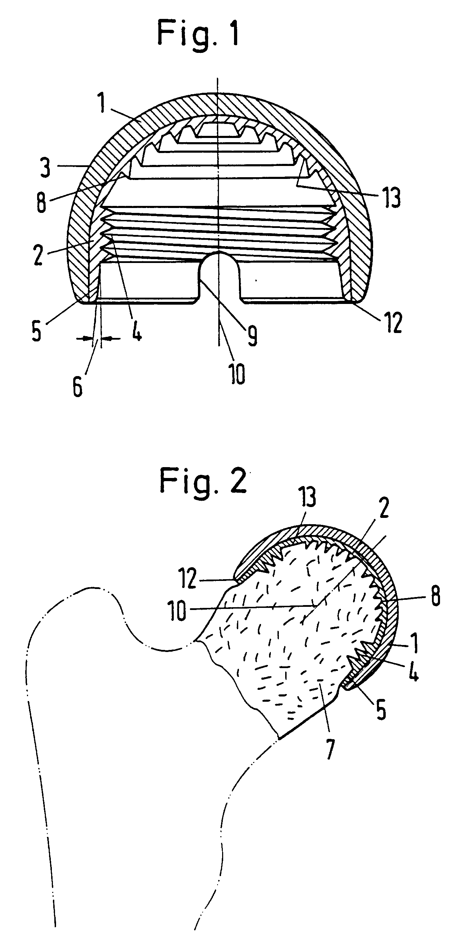

In Fig. 1 sind eine äussere Schale 3 aus einer Kobaltlegierung und eine innere Schale 2 aus Titan sich gegenseitig stützend miteinander verbunden. Die äussere Schale 3 besteht trotz Aussparungen 9, die die Fixierung in einem Setzwerkzeug dienen und die am Schalenrand 12 angebracht sind, aus einem mehr als halbkugelförmig zusammenhängenden Stück Kugelfläche, um für die Kräfteübertragung in verschiedenen Gelenkstellungen genügend grosse Uebertragungsflächen vorzusehen. Die Innenschale besitzt vom Schalenrand 12 her einen gegen innen konischen Einlauf 5, an den ein vorstehendes selbstschneidendes Innengewinde 4 anschliesst, während eine entsprechend der Aussenform gewölbte Innenfläche 13 den Abschluss bildet. Entsprechend dieser gewölbten Innenfläche 13 ist der Stumpf vom Femurkopf vorbearbeitet, wobei er bezüglich Aussendurchmesser so gross ist, dass die Kappe 1 beim Ansetzen mit dem konischen Einlauf 5 aufsitzt, der einen halben Konuswinkel 6 von ca. 5^{o} aufweist.

In Fig. 2 ist die Kappe 1 in implantiertem Zustand auf dem Stumpf eines Femurkopfes gezeigt. Die Kappe 1 wird mit einem Setzwerkzeug (nicht gezeigt), das ihr eine bezüglich Gewindeachse geführte Vorschubbewegung und über die Aussparungen 9 ein Schneidmoment überträgt, auf den Stumpf des Femurkopfes aufgeschraubt, bis die Bewegung durch das Auflaufen der gewölbten Innenfläche 13 auf dem Stumpf blockiert wird. Das Knochengewebe 7 unter der Kappe 1 wird somit an praktisch allen Berührungsflächen verspannt und erlaubt ohne Lockerung der Kappe die Uebertragung grosser Kräfte. Die Kappe 1 selbst ist durch das Gewinde 4 gegen das Lösen in Richtung Gewindeachse gesichert. Das Gewinde 4 ist selbsthemmend ausgeführt, was z.B. durch Unterbrüche im Gewinde erreicht wird, die sich mit Knochengewebe füllen. Zur Vergrösserung der Eingriffsfläche und zur zusätzlichen Aufnahme von Momentenanteilen des übertragenden Gelenkmoments sind auf der gewölbten Innenfläche 13 Schneidringe 8 angebracht, die zur Gewindeachse konzentrische Kreise bilden, und die in Richtung der Gewindeachse, die in diesem Beispiel mit der Polachse 10 zuzsammenfällt, scharfkantig vorstehen, um sich in das Knochengewebe 7 einzugraben.

Die Uebertragung der Gelenkkräfte auf das Knochengewebe des Femurkopfes findet innerhalb der kugelförmigen Kappe 1 statt. Der Femurhals, bei dem die grössten Biegemomente beim Uebergang zum Schaft auftreten, bleibt unberührt. Das mit dem Implantat in Berührung stehende Knochengewebe 7 steht unter Vorspannung und unter dem Stimulans der Kanten von Innengewinde 4 und Schneidringen 8 für das Einwachsen und die Bildung von frischem Knochengewebe. Mechanisch gesehen wird bereits mit der Primärverankerung die maximal zulässige Belastbarkeit erreicht.

## Patentansprüche

1. Zementfrei einbaubare Kappe (1) für einen Femurkopf bestehend aus einem Ausschnitt von einer Kugelschale, deren äussere Fläche mindestens einer halbkugelförmig zusammenhängenden Fläche entspricht, dadurch gekennzeichnet, dass die äussere Oberfläche (3) aus einer verschleissfesten Kobaltlegierung besteht, während die innere am Knochengewebe (7) angreifende Fläche (2) aus Titan besteht und zu einem selbstschneidenden Innengewinde (4) mit konischem Einläuf (5) geformt ist.

2. Kappe nach Anspruch 1, dadurch gekennzeichnet, dass die Innenfläche (2) in der Richtung der Gewindeachse (10) und konzentrisch zu der Gewindeachse (10) des Innengewindes (4) gegen das Knochengewebe (7) gerichtete Schneidringe (8) aufweist.

3. Kappe nach Anspruch 1 und 2, dadurch gekennzeichnet, dass die äussere Schalenwand Aussparungen (9) für die Aufnahme in einem Setzwerkzeug aufweist.

## Claims

1. A cap (1) for a femoral head, which can be fixed without the use of cement and consists of a segment of a ball socket, the outer surface of which corresponds at least to a hemispherical continuous surface,
**characterised in that** the outer surface (3) is made from a hard-wearing cobalt alloy, while the inner surface (2) bearing on the osseous tissue (7) is made from titanium and is formed with a conical insert (5) to produce a self-tapping internal screw thread (4).

2. A cap according to Claim 1,
**characterised in that** the inner surface (2) has cutting rings (8) directed towards the osseous tissue (7) in the direction of the thread axis (10) and concentrically to the thread axis (10) of the internal screw thread (4).

3. A cap according to Claim 1 and 2,
**characterised in that** the outer shell wall has recesses (9) for inclusion in a positioning tool.

## Revendications

1. Calotte (1) pour une tête de fémur, pouvant être installée sans ciment, composée d'un segment d'une coquille sphérique dont la face extérieure correspond au moins à une surface continue hémisphérique, caractérisée en ce que la surface extérieure (3) est réalisée à partir d'un alliage au cobalt résistant à l'usure, alors que la surface intérieure (2) entrant en contact avec le tissu osseux (7) est réalisée en titane et est configurée en un filetage intérieur auto-taraudeur (4) ayant une entrée conique (5).

2. Calotte selon la revendication 1, caractérisée en ce que la surface intérieure (2) comporte des anneaux de coupe (8) orientés, dans le sens de l'axe (10) du filetage (4), à l'encontre du tissu osseux (7), et disposés concentriquement à l'axe (10) du filetage.

3. Calotte selon les revendications 1 et 2, caractérisée en ce que la paroi extérieure de la coquille comporte des encoches (9) pour l'engagement dans un outil de pose.
